# FASCICULE DE BREVET EUROPEEN

(11) **EP 3 784 304 B1**
(45) Date de publication et mention de la délivrance du brevet: **25.05.2022**
(21) Numéro de dépôt: 19720975.2
(22) Date de dépôt: 05.04.2019
(51) Int. Cl.: A61L 27/54, C08L 5/16

(54) **DISPOSITIF IMPLANTABLE COMPRENANT UNE PIECE TEXTILE COMPRENANT DES FILS MULTIFILAMENTAIRES ET/OU DES FILS FILES DE FIBRES REVETU(E)S EN TOUT OU PARTIE D'UN POLYMERE DE CYCLODEXTRINE**
IMPLANTIERBARE VORRICHTUNG MIT EINER TEXTILKOMPONENTE AUS MULTIFILEN FÄDEN UND / ODER GESPONNENEN FÄDEN AUS FASERN, DIE VOLLSTÄNDIG ODER TEILWEISE MIT CYCLODEXTRINPOLYMER BESCHICHTET SIND
IMPLANTABLE DEVICE COMPRISING A TEXTILE COMPONENT COMPRISING MULTIFILAMENT THREADS AND/OR SPUN THREADS MADE OF FIBERS COMPLETELY OR PARTIALLY COATED WITH CYCLODEXTRIN POLYMER

(30) Priorité: 27.04.2018 FR 1853737
(43) Date de publication de la demande: 03.03.2021
(73) Titulaire: Cousin Biotech, 59117 Wervicq Sud (FR)
(72) Inventeur: VERMET, Guillaume, 69008 LYON (FR); MARTEL, Bernard, 59850 NIEPPE (FR); BLANCHEMAIN, Nicolas, 59660 MERVILLE (FR); DEGOUTIN, Stéphanie, 59310 LANDAS (FR)
(74) Mandataire: Cabinet Beau de Loménie
(86) Numéro de dépôt international: PCT/FR2019/050803
(87) Numéro de publication internationale: WO 2019/207227

(56) Documents cités:
- WO-A1-2006/051227
- G. VERMET ET AL: "Visceral mesh modified with cyclodextrin for the local sustained delivery of ropivacaine", INTERNATIONAL JOURNAL OF PHARMACEUTICS, vol. 476, no. 1-2, 30 septembre 2014 (2014-09-30), pages 149-159, XP055550035, NL ISSN: 0378-5173, DOI: 10.1016/j.ijpharm.2014.09.042 cité dans la demande

## Description

### Arrière-plan de l'invention

La présente invention concerne le domaine technique des dispositifs implantables comprenant une pièce textile comprenant des fils multifilamentaires et/ou des fils filés de fibres au moins partiellement revêtus d'un polymère de cyclodextrine.

Les dispositifs implantables comprenant une pièce textile peuvent être utilisés dans de nombreuses applications, par exemple dans le domaine du rachis, pour le traitement d'une hernie abdominale, en remplacement d'un ligament, pour le traitement des prolapsus des organes pelviens (par exemple en traitement de l'incontinence urinaire), pour le traitement des troubles érectiles masculins, pour le traitement de l'obésité ; et sous des formes très variées : pièce plane ou en trois dimensions, par exemple sous forme d'un dôme, d'une cale, d'une tresse, ou de bandelettes.

Suite à la pose d'un dispositif implantable, des complications, telles que des adhérences, une douleur, et des infections post-opératoires, peuvent survenir et retarder la guérison et le retour du patient à une activité normale, en particulier s'agissant de la cure des hernies abdominales.

De plus, la douleur post-opératoire dans les premiers jours suite à une intervention chirurgicale a récemment été liée à l'apparition de douleurs chroniques, plusieurs mois après l'intervention chirurgicale, résultant en une morbidité qui peut nécessiter un acte de traitement additionnel.

De telles conséquences peuvent être empêchées par un traitement prophylactique consistant dans l'injection locale de dérivés de morphine ou l'instillation continue d'anesthésiques en local après l'implantation.

Cependant, de nombreux protocoles d'analgésie post-opératoire par infiltration directe dans le site opéré ou instillation continue d'anesthésiques locaux ont été proposés sans atteindre un compromis satisfaisant entre la méthode de traitement de la douleur et le soulagement effectif de cette dernière. En effet, l'effet analgésique attendu et durable ne peut être obtenu du fait de l'élimination rapide du médicament du site opéré par le flux circulant des fluides corporels.

Une autre approche consiste à modifier les dispositifs implantables en sorte de leur conférer des propriétés de délivrance d'agent(s) actif(s), tels que des analgésiques, in-situ et de manière plus ou moins prolongée selon le dispositif de libération retenu.

Parmi ces dispositifs de libération in-situ, on connaît les polymères de cyclodextrine appliqués à un textile en sorte de former un revêtement hôte qui sera fonctionnalisé par ajout d'un ou plusieurs agent(s) actif(s) invité(s). WO 2006/051227 A1 décrit ainsi la fabrication d'un biomatériau pouvant être en textile et comprenant un polymère de cyclodextrine. L'agent actif complexé est choisi en fonction de son aptitude à être complexé avec les molécules cages de cyclodextrine et selon l'activité thérapeutique souhaitée. Il peut ainsi s'agir d'un antibiotique afin d'éviter toute contamination bactérienne au niveau de la prothèse implantée.

La publication intitulée « Visceral mesh modified with cyclodextrin for the local sustained delivery of ropivacaine », International Journal Of Pharmaceutics 476 (2014) 149-159 (auteurs : G.Vermet, S.Degoutin, F.Chai, M.Maton, M.Bria, C.Danel, H.F.Hildebrand, N.Blanchemain, B.Martel) décrit également un dispositif de libération in-situ appliqué à une plaque textile en polyester comprenant un polymère de cyclodextrine complexant de la ropivacaine afin de réduire la douleur post-opératoire.

De manière générale, les textiles sont utilisés en tant que matériaux implantables car leur souplesse leur permet de s'adapter à des formes complexes, en particulier à la forme anatomique des organes.

Selon les propriétés recherchées, les textiles peuvent être rigides ou semi-rigides ou souples. Ces textiles implantables peuvent être enroulés sur eux-mêmes lorsqu'ils sont souples et sous la forme de panneaux plans ou encore suivre des trajets anatomiques complexes lorsqu'ils sont sous la forme de tresses ou assurer des fonctions spécifiques, tel que le comblement selon leur configuration en 3D.

Les pièces textiles implantables comprenant des fils monofilamentaires sont préférées car considérées comme étant moins propices au développement d'infection bactérienne comparativement aux pièces textiles implantables comprenant des fils multifilamentaires (cf. J. Conze, R. Rosch, U. Klinge, C. Weiss, M. Anurov, S. Titkowa, A. Oettinger, V. Schumpelick, Polypropylene in the intra-abdominal position: influence of pore size and surface area, Hernia 8 (2004) 365-372). Cependant, il est difficile de fixer un revêtement polymère sur ces pièces textiles constituées de fils monofilamentaires

Avantageusement, les pièces textiles comprenant des fils multifilamentaires possèdent une surface spécifique et une porosité supérieures à celles des pièces textiles comprenant des fils monofilamentaires ainsi que davantage d'espaces interfilamentaires confinés au cœur du fil pouvant être comblés par un revêtement polymère.

De plus, le dépôt d'un revêtement polymère sur un textile, selon sa nature, peut modifier la souplesse du textile à un point que ce dernier ne peut plus assurer correctement ses fonctions premières. C'est le cas par exemple s'agissant des prothèses de réfection de la paroi abdominale lorsque la prothèse ne peut plus être enroulée sur elle-même pour être insérée dans un trocart (notamment ayant un diamètre de l'ordre de 10 mm ou est trop épaisse à l'état enroulé.

Ce paramètre peut être rédhibitoire pour l'utilisation d'une pièce textile implantable fonctionnalisée puisque pour que le revêtement polymère hôte soit efficace, il faut qu'il soit déposé en quantité suffisante sur la pièce textile afin d'y absorber une quantité d'agent(s) actif(s) qui corresponde à une dose thérapeutique délivrée efficace mais qui ne soit pas toxique. Cette dose d'actif absorbée doit donc être comprise dans la fenêtre thérapeutique à savoir entre une valeur minimale (seuil d'efficacité), et une valeur maximale (seuil de toxicité).

La présente invention a ainsi pour objet un dispositif implantable comprenant une pièce textile revêtue au moins en partie par un polymère de cyclodextrine conservant ses propriétés mécaniques intrinsèques, notamment sa souplesse.

La présente invention a pour objet également un dispositif implantable dont la durée et l'intensité du ou des effets associés à un plusieurs actif(s) complexé(s) (par exemple analgésique et/ou antibactérien) sont améliorées.

### Objet et résumé de l'invention

La présente invention a pour objet, selon un premier aspect, un dispositif implantable, palliant les problèmes précités, comprenant une pièce textile comprenant des fils multifilamentaires et/ou des fils filés de fibres au moins partiellement revêtu(e)s d'un revêtement polymère hôte, ledit revêtement polymère hôte comprend un polymère de cyclodextrine(s) et/ou dérivés de cyclodextrine(s) et/ou de complexe(s) d'inclusion de cyclodextrine et/ou de complexe(s) d'inclusion de dérivés de cyclodextrines. Avantageusement, au moins une partie des filaments et/ou des fibres ont chacun(e) un diamètre inférieur ou égal à 25 micromètres (µm).

Malgré les préjugés de l'homme de l'art, les inventeurs ont trouvé que la combinaison d'un revêtement polymère hôte avec une pièce textile ayant des filaments et/ou des fibres d'une certaine finesse permet d'obtenir un implant souple et pouvant être plié sans que le revêtement ne se détache de la fibre ou du filament lors de la manipulation de l'implant. Cette combinaison permet en outre d'améliorer le taux de greffage en polymère hôte et donc d'améliorer encore l'effet thérapeutique recherché du fait d'un chargement en agent(s) actif(s) également amélioré.

Avantageusement, les pièces textiles comprenant des fils multifilamentaires, et/ou des fils filés de fibres, notamment des fils multifilamentaires, possèdent une surface spécifique et une porosité supérieures à celles des pièces textiles comprenant des fils monofilamentaires ainsi que davantage d'espaces interfilamentaires confinés au cœur du fil pouvant être comblés par un revêtement polymère.

On comprend par pièce textile dans le présent texte toute pièce obtenue par la mise en œuvre d'au moins un fil, en particulier le ou lesdits fil(s) peut/peuvent être un fil multifilamentaire et/ou un fil filé de fibres.

La pièce textile selon l'invention comprend des fils multifilamentaires mais peut comprendre également des fils filés de fibres, bien que cela ne soit pas préféré.

La masse volumique du polyéthylène téréphtalate est considérée comme étant de préférence de l'ordre de 1,38 g/cm³ et celle du polypropylène est considérée comme étant de préférence de l'ordre de 0,9 g/cm³.

Le diamètre d'un filament ou d'une fibre est calculé de préférence en considérant que le filament ou la fibre est un cylindre parfait à partir de la formule suivante : diamètre du filament ou de la fibre [µm] = 20 ^{∗} Racine ²(Titre [dtex)] filament ou de la fibre / (masse volumique du matériau constituant la fibre ou le filament [g/cm³] × 1/3,14)).

La pièce textile selon l'invention peut être un élément tricoté, notamment à mailles cueillies (i.e tricots trames) ou à mailles jetées (i.e tricots chaines), un élément tissé, un élément tressé, un élément retordu ou câblé, ou résultant de leurs différentes combinaisons.

Un fil multifilamentaire comprend un ensemble de plusieurs filaments. Le fil multifilamentaire est manipulé mécaniquement sur un métier de fabrication d'un élément textile, par exemple un métier à tricoter ou à tisser ou à tresser.

Les fibres selon l'invention peuvent être sous forme d'un fil filé de fibres

De préférence, la pièce textile est dans un matériau textile comprenant des première et seconde faces opposées, encore de préférence le revêtement polymère hôte revêt tout ou partie de la première face et/ou de la seconde face.

Dans un mode de réalisation, une partie ou la totalité des filaments des fils multifilamentaires et/ou des fibres des fils filés de fibres ont un diamètre inférieur ou égal à 20 µm, de préférence inférieur ou égal à 18 µm, encore de préférence inférieur ou égal à 17 µm, en particulier supérieur ou égal à 5 µm.

Dans un mode de réalisation, la masse du revêtement polymère hôte (mh), notamment sa masse surfacique (g/m²), par rapport à la masse (g) totale (mt), notamment la masse surfacique totale (g/m²), de la pièce textile comprenant le revêtement polymère hôte (mh/mt ^{∗}100), est supérieur à 0% et inférieur ou égal à 60%, de préférence inférieur ou égal à 50%, notamment inférieur ou égal à 40%, en particulier supérieur ou égal à 5%, plus particulièrement supérieur ou égal à 10%.

Dans un mode de réalisation, le taux de greffage en revêtement polymère hôte, notamment en polymère de cyclodextrine défini selon l'invention, est supérieur ou égal à 10%, de préférence supérieur ou égal à 15%, encore de préférence supérieur ou égal à 20%, en particulier supérieur ou égal à 25%, plus particulièrement supérieur ou égal à 30%.

Le taux de greffage en revêtement polymère hôte peut être inférieur ou égal à 60%.

Le taux de greffage est calculé comme étant le gain en masse (exprimé en pourcentage) de la pièce textile en revêtement polymère hôte. Ce taux de greffage est égal à : ((masse pièce textile y compris revêtement polymère hôte) - (masse pièce textile vierge i.e hors revêtement polymère hôte))/(masse pièce textile vierge i.e hors revêtement polymère hôte)^{∗}100.

Dans le cadre de la présente invention, la masse de la pièce textile vierge ou revêtue du revêtement polymère hôte est de préférence mesurée après un séchage à 104°C pendant une heure (et éventuellement après l'étape de neutralisation et/ou l'étape de lavage définie(s) ci-après), et son placement dans un dessiccateur à température ambiante (de l'ordre de 20°C-25°C) pendant au moins 30 minutes.

Dans un mode de réalisation, au moins une partie, notamment la totalité, des fils filés de fibres et/ou des fils multifilamentaires ont chacun un titre inférieur ou égal à 500 dtex, de préférence inférieur ou égal à 400 dtex, encore de préférence inférieur ou égal à 300 dtex, plus préférentiellement inférieur ou égal à 200 dtex, en particulier inférieur ou égal à 100 dtex.

Dans un mode de réalisation, au moins une partie des filaments et/ou des fibres des fils, notamment les filaments des fils multifilamentaires ont chacun(e) un titre inférieur ou égal à 10 dtex (et implicitement supérieur à 0 dtex), de préférence inférieur ou égal à 5 dtex, encore de préférence inférieur ou égal à 4 dtex, notamment inférieur ou égal à 3 dtex.

Dans un mode de réalisation, la pièce textile (hors revêtement polymère hôte) a une masse surfacique supérieure ou égale à 10 g/m² et inférieure ou égale à 500 g/m², de préférence inférieure ou égale à 300 g/m², encore de préférence ou égale à 120 g/m², préférentiellement inférieure ou égale 80 g/m², plus préférentiellement inférieure ou égale à 60 g/m², notamment inférieure ou égale à 40 g/m², par exemple de l'ordre de 38 g/m².

Dans un mode de réalisation, la pièce textile est plane ou en trois dimensions, de préférence la pièce textile a une forme sensiblement de plaque, éventuellement lisse ou en reliefs.

Le polymère de cyclodextrine présent sur la pièce textile est formé par une réaction de réticulation entre la cyclodextrine ou un dérivé de la cyclodextrine et au moins un agent réticulant défini ci-après. La cyclodextrine et/ou un dérivé de cyclodextrine et ledit au moins un agent réticulant sont mélangés en solution aqueuse puis la pièce textile est imprégnée de ladite solution aqueuse par exemple par foulardage. Cette opération d'imprégnation, en particulier par foulardage, permet à la cyclodextrine et/ou au dérivé de la cyclodextrine et audit au moins un agent réactif de pénétrer par capillarité à l'intérieur des interstices formés entre les filaments des fils et/ou entre les fibres. Le polymère de cyclodextrine et/ou dérivé de cyclodextrine comble ainsi l'espace interfilamentaire et/ou interfibreux et la surface externe du fil et/ou des fibres.

Avantageusement, les fils et/ou les fibres selon l'invention présentent une surface spécifique et un taux d'absorption de la solution aqueuse lors de l'imprégnation importants. Le taux de greffage en polymère de cyclodextrine et/ou dérivé de cyclodextrine est ainsi amélioré. Etonnamment, la pièce textile conserve sa souplesse intrinsèque.

On entend par « cyclodextrine » dans le présent texte, toute cyclodextrine native, en particulier l'alpha-cyclodextrine ou la beta-cyclodextrine ou la gamma-cyclodextrine.

On entend par « dérivé de cyclodextrine » dans le présent texte, toute cyclodextrine native, en particulier l'alpha-cyclodextrine ou la beta-cyclodextrine ou la gamma-cyclodextrine, dont un ou plusieurs groupe(s) hydroxyle(s), est/sont substitué(s), en particulier aminé(s), notamment par une fonction amine (-NH₂), estérifié(s), alkylé(s), hydroxyalkylé(s), carboxyalkylé(s), en particulier carboxyméthylé(s), sulfoalkylé(s), en particulier sulfobutylé(s).

Ledit au moins un groupe hydroxyle de la cyclodextrine peut être substitué par un chaîne alkyle, linéaire et/ou ramifiée, insaturée ou non, substituée ou non, comprenant de 1 à 10 atomes de carbone, de préférence de 1 à 6 atomes de carbones, encore de préférence de 1 à 4 atomes de carbone, notamment un groupe méthyle, un groupe éthyle, un groupe n-propyl, un groupe isopropyl, un groupe n-butyl, un groupe iso-butyl, un groupe sec-butyl, un groupe ter-butyle, chacun desdits groupes étant éventuellement, et ce indépendamment les uns des autres, substitué(s) par un ou plusieurs groupes hydroxyles (-OH) ; et/ou substitué(s) par un ou plusieurs groupes acides carboxyliques (-COOH) ; et/ou substitué(s) par un ou plusieurs groupes acides sulfoniques et/ou un ou des sels desdites fonctions acides sulfoniques et carboxyliques. Ledit au moins un groupe hydroxyle de la cyclodextrine peut ainsi être substitué par un groupe hydroxypropyl ou par un groupe carboxyméthyl.

On entend par « complexe d'inclusion de cyclodextrine » ou « complexe d'inclusion de dérivé de cyclodextrine » dans le présent texte, toute cyclodextrine ou dérivé de cyclodextrine au sens de la présente invention complexant un agent fonctionnel tel que défini dans le présent texte.

L'homme du métier sélectionne une ou plusieurs cyclodextrine(s) et/ou un ou plusieurs dérivé(s) de cyclodextrine et/ou un ou plusieurs complexe(s) d'inclusion (de dérivés) de cyclodextrine apte(s) à réagir avec l'agent réticulant. En particulier, si un ou des dérivés de cyclodextrine est/sont utilisé(s), il(s) doi(ven)t présenter suffisamment de groupes hydroxyles (-OH) pour être apte(s) à effectuer une réaction de polycondensation avec les acides (poly)carboxyliques et/ou leurs anhydrides correspondants.

EP 1 165 621 B1 décrit la synthèse de polymères de cyclodextrine obtenus par réactions de polycondensation entre des acides (poly)carboxyliques et les cyclodextrines natives (alpha, béta, gamma) ou dérivées (méthyl, hydroxypropyl cyclodextrines) convenant parfaitement à la mise en œuvre de l'invention.

Ils se caractérisent par une forte teneur en cyclodextrine, comprise entre 40% et 65% en masse et par sa richesse en fonctions acides carboxyliques, de l'ordre de 3-5 mmol par gramme de polymère de cyclodextrine.

On comprend au sens de la présente invention par « acide carboxylique » un acide comprenant une fonction -COOH.

On comprend au sens de la présente invention par « acide polycarboxylique », un acide comprenant au moins deux fonctions -COOH, et par « son anhydride correspondant », un anhydrideformé à partir de la condensation de deux fonctions acides carboxyliques, en particulier comprenant au moins une fonction -CO-O-CO-.

Dans une variante, le rapport de la masse du revêtement hôte sur la masse totale de la pièce textile comprenant le revêtement hôte est supérieur ou égal à 10%, de préférence supérieur ou égal à 12%, encore de préférence supérieur ou égal à 15%.

Dans une sous-variante, ce rapport est supérieur ou égal à 20%, en particulier supérieur ou égal à 25%.

Dans une variante, la pièce textile (hors revêtement polymère hôte) a une masse surfacique inférieure ou égale à 120 g/m², de préférence inférieure ou égale à 80 g/m², encore de préférence inférieure ou égale à 70 g/m², encore de préférence inférieure ou égale à 50 g/m².

Dans une variante, la pièce textile comprend, de préférence dans une proportion massique d'au moins 80% par rapport à sa masse totale, encore de préférence dans une proportion massique d'au moins 95% par rapport à sa masse totale, des fils multifilamentaires et/ou des fils filés de fibres dont le titre de chacun des filaments est inférieur ou égal à 10 dtex, de préférence inférieur ou égal à 7 dtex, encore de préférence inférieur ou égal à 4 dtex.

Dans une variante, la pièce textile est une pièce tricotée, notamment à mailles jetées.

De préférence, la pièce textile est un tricot indémaillable.

Dans une variante, le dispositif selon l'invention est choisi dans la liste constituée d'une prothèse pour le traitement d'une hernie dans la région abdominale, notamment d'une hernie inguinale, fémorale, ombilicale ou épigastrique ; d'un ligament ; d'une prothèse en traitement d'un prolapsus d'au moins un organe pelvien, d'une prothèse de traitement des dysfonctionnements érectiles masculins ; d'une prothèse pour le traitement du rachis, notamment d'une prothèse inter-vertébrale ; de préférence d'une prothèse pour le traitement d'une hernie dans la région abdominale.

Dans une variante, les fils multifilamentaires et/ou les fils filés de fibres sont dans au moins un ou plusieurs polymère(s) résorbable(s), partiellement résorbable(s) ou non résorbable(s), en particulier dans un ou plusieurs polymère(s) choisi(s) dans la liste I constituée par : les polyoléfines, et notamment le polypropylène et le polyéthylène ; les polymères à base de polyoxyde d'éthylène (PEO), les copolymères à blocs à base de polyoxyde de propylène et d'oxyde d'éthylène, notamment de type dibloc ou tribloc, tels que le PPO-PEO (i.e le poly(propylene oxyde-ethylene oxide), le PPO-PEO-PPO (ie le poly(propylene oxyde-ethylene oxyde-propylene oxyde) ou le PEO-PPO-PEO ( i.e le poly(éthylène oxyde-propylène oxyde-éthylène oxyde)), les polyesters (en particulier non résorbables), et notamment le polyéthylène téréphtalate ; les polyamides, et notamment le polyamide 6-6 ; encore de préférence il s'agit du polypropylène ou de polyéthylène téréphtalate ; et/ou dans la liste II constituée par : les (co)polymères d'acide lactique, issu(s) de la polymérisation au moins du L Lactide et/ou au moins du D Lactide et/ou au moins du mésolactide, ; les polymères issus de la polymérisation au moins du glycolide, par exemple l'acide polyglycolique (PGA) ; les copolymères issus de la polymérisation d'au moins du lactide et d'au moins du glycolide, par exemple le PLGA (polylactide-co-glycolide) ; les (co)polymères à blocs à base de polylactide et de polyéthylèneglycol, par exemple de type PLA-PEG-PLA ; ou un mélange de ces derniers.

Les (co)polymères d'acide lactique définis ci-dessus sont communément désignés sous le terme générique de polymère d'acide polylactique (PLA).

Dans une variante, le revêtement polymère hôte comprend au moins un agent fonctionnel invité choisi parmi la liste comprenant : les antibiotiques, par exemple la ciprofloxacine ; les agents anti-inflammatoires ; les anticoagulants ; les anti-thrombogéniques ; les agents anti-mitotiques ; les agents anti-prolifération ; les agents anti-adhésion ; les agents anti-migration ; les promoteurs d'adhésion cellulaire ; les facteurs de croissance ; les molécules antiparasitaires ; les hormones ; les antifongiques ; les molécules antimicrobiennes ; les antiseptiques ; les agents analgésiques tels que la ropivacaine, la bupivacaine, la lidocaine, la lévobupivacaine, de préférence la ropivacaine.

Dans une variante, la pièce textile comprend des ouvertures, en particulier comprend des ouvertures ayant au moins une dimension supérieure ou égale à 1 mm.

Ces ouvertures sont de préférence traversantes, c'est-à-dire qu'elles traversent l'épaisseur du matériau textile formant la pièce textile et débouchent sur ses première et seconde faces.

Ces ouvertures sont de préférence délimités entre les fils, notamment de manière répétée selon le schéma de mailles ou l'armure de tissage par exemple.

Avantageusement, la pièce textile permet le développement de la fibrose et son intégration dans les tissus vivants par l'intermédiaire de ces ouvertures.

Dans une variante, la pièce textile est choisie dans la liste constituée par : les tricots à mailles jetées ou à mailles cueillies ; les tissus, les tresses, les éléments retordus, ou leur combinaison.

La présente invention a pour objet, selon un second aspect, un procédé de fabrication d'un dispositif implantable, de préférence selon l'une quelconque des variantes de réalisation en référence à un premier aspect de l'invention, comprenant les étapes suivantes :
- (i) fourniture d'une pièce textile comprenant des fils multifilamentaires et/ou des fils filés de fibres, au moins une partie des filaments et/ou des fibres ont chacun(e) un diamètre inférieur ou égal à 25 micromètres (µm) ;
- (ii) application d'une solution aqueuse comprenant :
   - au moins une cyclodextrine et/ou au moins un dérivé de cyclodextrine et/ou au moins un complexe d'inclusion de cyclodextrine et/ou au moins un complexe d'inclusion de dérivé de cyclodextrine ;
   - au moins un acide (poly)carboxylique et/ou son anhydride d'acide (poly)carboxylique,
   - et éventuellement au moins un catalyseur ;
   sur au moins une partie des fils multifilamentaires et/ou des fils filés de fibres ;
- (iii) application d'un traitement thermique à la pièce textile obtenue après l'étape (ii) afin de former un revêtement hôte comprenant un polymère de cyclodextrine(s) et/ou dérivés de cyclodextrine(s) et/ou de complexe(s) d'inclusion de cyclodextrine et/ou de complexe(s) d'inclusion dérivés de cyclodextrines ;
- obtention du dispositif implantable.

De préférence, la solution aqueuse est appliquée par foulardage.

Dans une variante, l'acide (poly)carboxylique ou son anhydride d'acide correspondant est choisi dans la liste comprenant : les acides (poly)carboxyliques saturés ou insaturés ou aromatiques, linéaires ou branchés ou cycliques et les acides hydroxypoly(carboxyliques), de préférence dans la liste comprenant : l'acide citrique, l'acide polyacrylique, l'acide poly(méthacrylique), l'acide 1,2,3,4-butanetétracarboxylique (BTCA), l'acide maléique, l'acide citraconique, l'acide itaconique, l'acide 1,2,3-propanetricaboxylique, l'acide trans-aconitique, l'acide all-cis-1,2,3,4-cydopentanetétracarboxylique, l'acide méllitique, l'acide pyroméllitique, l'acide éditique (acide éthylene diamine tétraacétique ou EDTA), l'acide oxydisuccinique, l'acide thiodisuccinique ou parmi les anhydrides acides desdites acides (poly)carboxyliques précités, tel que le dianhydride pyroméllitique, et leurs mélanges, de préférence l'acide citrique et le BTCA.

Dans un mode de réalisation, le traitement thermique à l'étape (iii) comprend l'application d'une température supérieure ou égale à 80°C, de préférence une température supérieure ou égale à 90°C, encore de préférence supérieure ou égale 100°C, notamment supérieure ou égale à 130°C, pendant au moins une minute, encore de préférence pendant au moins 5 minutes.

Dans un mode de réalisation, le traitement thermique à l'étape (iii) comprend une première étape (iii-a) au-cours de laquelle, on chauffe à une température déterminée en sorte d'évaporer l'eau, en particulier sans induire de polymérisation, et une seconde étape (iii-b) au-cours de laquelle on chauffe à une température déterminée en sorte de faire polymériser le revêtement polymère hôte.

De préférence, la température lors de la première étape (iii-a) est supérieure ou égale à 90°C, de préférence pendant au moins dix minutes.

De préférence, la température lors de la seconde étape (iii-b) est supérieure ou égale à 120°C, encore de préférence supérieure ou égale à 135°C, notamment pendant au moins trente minutes, en particulier pendant au moins deux heures.

La température appliquée lors des étapes (iii-a) et (iii-b) est inférieure à la température de dégradation de la pièce textile, de préférence inférieure ou égale à 250°C, encore de préférence inférieure ou égale à 180°C.

Le temps d'application lors des étapes (iii-a) et (iii-b) est de préférence inférieur ou égal à 360 minutes.

De préférence, le procédé de fabrication comprend une étape de lavage (iv) de la pièce textile après l'étape (iii) (en particulier après l'étape (iii-b) et/ou l'étape de neutralisation ci-après) au-cours de laquelle la pièce textile est lavée avec de l'eau, notamment de l'eau distillée ou de l'eau pure, ou une solution aqueuse, ayant une température supérieure ou égale à 45°C, notamment supérieure ou égale à 70°C.

Cette opération permet d'ôter les réactifs présents dans la solution aqueuse n'ayant pas agi.

Dans une variante, le procédé comprend une étape de neutralisation de l'acidité résiduelle du revêtement hôte, de préférence on applique une solution basique, en particulier une solution de carbonate de sodium ou d'hydrogénocarbonate de sodium (bicarbonate), à la pièce textile obtenue après l'étape (iii) (en particulier l'étape (iii-b).

De préférence, un lavage intermédiaire (similaire au lavage de l'étape (iv)) est effectué après l'étape (iii) (notamment (iii-b) et avant l'étape de neutralisation, encore de préférence avant l'étape (iv).

Ce traitement au moyen d'une base faible provoque la conversion des fonctions acides carboxyliques résiduelles du revêtement du polymère de cyclodextrine en fonctions carboxylates. Avantageusement, les inventeurs ont constaté de manière surprenante que cette étape permet d'assouplir la pièce textile revêtue du polymère hôte.

Cette étape permet également d'améliorer la cytocompatibilité du dispositif implantable.

Avant implantation, le dispositif implantable est stérilisé, par exemple dans son sachet d'emballage par irradiation ou à l'oxyde d'éthylène.

### Description détaillée de l'invention

La présente invention sera mieux comprise à la lecture des exemples de réalisation décrits ci-après et cités à titre non limitatif.

### Matériels utilisés :

Pièce textile A : panneau plan de 38 g/m² tricoté à mailles jetées constitué de fils multifilamentaires en polyéthylène téréphtalate, lesdits fils ayant chacun un titre de 61 dtex et comprenant chacun 24 filaments (soit environ 2,54 dtex/filament, soit environ un diamètre de 15,3 µm/filament).

Pièce textile B : panneau plan de 100 g/m² tricoté à mailles jetées constitué de fils multifilamentaires en polyéthylène téréphtalate, les fils de chaine (70% en nombre), notamment formant la chaînette, ayant chacun un titre de 138 dtex et comprenant chacun 32 filaments (soit environ 4,31 dtex, soit environ un diamètre de 20 µm/filament) ; et les fils de trame (30% en nombre) ayant chacun un titre de 226 dtex, et comprenant chacun 64 filaments (soit environ 3,53 dtex, soit environ un diamètre de 18 µm/filament. Le diamètre moyen des filaments est ainsi environ de 19,5 µm.

Hydroxypropyl-β-cyclodextrine (HPβCD), de la marque Kleptose^{®} HPB, MS= 0,62 ; M = 1387 g/mol de Roquette Frères (Lestrem, France).

Acide citrique (CTR), Sodium dihydrogen hypophosphite (NaH₂PO₄.xH₂O) et ropivacaine (M= 274 g/mol= de Aldrich Chemicals (Saint Quentin Fallavier, France).

Solution de chlorhydrate de ropivacaine : Naropin^{®} 2mg/ml et Ropivacaine KABI 10mg/ml, respectivement de AstraZeneca (Londres, Angleterre) et de Kabi (Bad Homburg, Allemagne).

Les pièces textiles A et B sont au préalables lavées par extraction au soxhlet avec du diéthyl éther (24 cycles, une heure par cycle) dans le but d'éliminer des lubrifiants et d'éventuels composés résiduels, par exemple des agents d'ensimage.

### Adsorption/Désorption de la ropivacaine

Après fonctionnalisation des pièces textiles par le polymère de cyclodextrine, les pièces textiles sont imprégnées dans une solution de chlorhydrate de ropivacaine à température ambiante (de l'ordre de 20-25°C). Afin d'évaluer la quantité de chlorhydrate de ropivacaine adsorbée par les pièces textiles, la ropivacaine adsorbée est désorbée dans 10 ml d'eau puis dans 5 ml de soude NaOH à 0,1M. Les solutions de désorption obtenues sont analysée en utilisant une méthode de chromatographie en phase liquide haute performance (LC 2010A-HT, Shimadzu, Noisiel, France, équipée avec un détecteur visible-ultraviolet et un ordinateur Dell intégrateur/enregistreur avec le logiciel LabSolutions). Le chromatographe a été adapté avec une colonne Gemini-NX C18 (5 µm, 250 ^{∗} 4.6 mm, Phenomenex, Le Pecq, France). La phase mobile consiste dans un mélange d'une solution tampon de phosphate à 72% (pH 2.5) et 28% d'acétonitrile. La séparation chromatographique a été effectuée à 25°C avec un débit de 1 mL/min et contrôlé à 201 nm. Le temps de rétention approximatif pour la ropivacaine à 20 nm est de 5,5 minutes dans ces conditions.

La quantité de ropivacaine désorbée dans l'eau ultrapure correspond à la ropivacaine adsorbée par capillarité sur la pièce textile elle-même, « indépendamment » du revêtement polymère hôte (interactions non spécifiques).

La quantité de ropivacaine désorbée dans la soude NaOH à 0,1M correspondant à la ropivacaine chargée par le revêtement polymère hôte (interactions spécifiques avec le polymère de cyclodextrine : complexes d'inclusion, liaisons ioniques liaisons hydrogènes).

Exemple 1 : La pièce textile A est imprégnée et foulardée (2m/min, 2 bars, Laboratory Padder LDP, LAB-PRO, Wikon, Switzerland) dans une solution aqueuse comprenant HPβCD, un catalyseur (NaH₂PO₂) et CTR, dans les proportions suivantes 8/1/10 dans lesquelles 8, 1 et 10 sont les masses (gramme) de HPβCD, du catalyseur (NaH₂PO₂) et de CTR, respectivement, dissous dans 100 mL d'eau ultrapure. La pièce textile A imprégnée est placée dans une enceinte de thermofixation (Modèle FD, BINDER, Tuttlingen, Allemagne) pendant 5 minutes à 90°C puis pendant 120 minutes à 140°C. La pièce textile A revêtue est ensuite lavée deux fois rapidement 3 minutes dans l'eau ultrapure puis neutralisée par passage dans une solution aqueuse comprenant du carbonate de sodium à 4 g/L afin de neutraliser l'acidité résiduelle. La pièce textile est ensuite rincée deux fois rapidement dans l'eau ultrapure pendant 3 minutes. La pièce textile est ensuite lavée dans l'eau ultrapure (étape de lavage) à 80°C (FISONS C1, Thermo Scientific Haake, Karlsruhe, Allemagne) pendant 20 minutes et séchée pendant 104°C pendant 1 heure, et enfin refroidie 30 minutes à température ambiante dans un dessicateur. Le taux de greffage en revêtement hôte est de l'ordre de 13,9 %. La pièce textile A finale est souple, et s'enroule sur elle-même sans altération de sa forme, ni détachement du revêtement polymère de CD.

Exemple 2: La pièce textile A est imprégnée et foulardée (2m/min, 2 bars, Laboratory Padder LDP, LAB-PRO, Wikon, Switzerland) dans une solution aqueuse comprenant HPβCD, un catalyseur (NaH₂PO₂) et CTR, dans les proportions suivantes 12/1,5/15 dans lesquelles 12 ; 1,5 et 15 sont les masses (grammes) de HPβCD, du catalyseur (NaH₂PO₂) et de CTR, respectivement, dissous dans 100 mL d'eau ultrapure. La pièce textile A imprégnée est placée dans une enceinte de thermofixation (Modèle FD, BINDER, Tuttlingen, Allemagne) pendant 5 minutes à 90°C puis pendant 120 minutes à 140°C. La pièce textile A revêtue est ensuite lavée deux fois rapidement 3 minutes dans l'eau ultrapure puis neutralisée par passage dans une solution aqueuse comprenant du carbonate de sodium à 4 g/L afin de neutraliser l'acidité résiduelle. La pièce textile est ensuite rincée deux fois rapidement dans l'eau ultrapure pendant 3 minutes. La pièce textile est ensuite lavée dans l'eau ultrapure à 80°C (FISONS C1, Thermo Scientific Haake, Karlsruhe, Allemagne) pendant 20 minutes et séchée pendant 104°C pendant 1 heure, et enfin refroidie 30 minutes à température ambiante dans un dessicateur. Le taux de greffage est revêtement hôte est de l'ordre de 21 %. La pièce textile A finale est souple, et s'enroule sur elle-même sans altération de sa forme ni détachement du revêtement polymère hôte de CD.

Exemple 3 : La pièce textile A est imprégnée et foulardée (2m/min, 2 bars, Laboratory Padder LDP, LAB-PRO, Wikon, Switzerland) dans une solution aqueuse comprenant HPβCD, un catalyseur (NaH₂PO₂) et CTR, dans les proportions suivantes 16/2/20 dans lesquelles 16, 2 et 20 sont les masses (grammes) de HPβCD, du catalyseur (NaH₂PO₂) et de CTR, respectivement, dissous dans 100 mL d'eau pure. La pièce textile A imprégnée est placée dans une enceinte de thermofixation thermofixation (Modèle FD, BINDER, Tuttlingen, Allemagne) pendant 5 minutes à 90°C puis pendant 120 minutes à 140°C. La pièce textile A revêtue est ensuite lavée deux fois rapidement 3 minutes dans l'eau ultrapure puis neutralisée par passage dans une solution aqueuse comprenant du carbonate de soude concentrée à 4 g/L afin de neutraliser l'acidité résiduelle. La pièce textile est ensuite rincée deux fois rapidement dans l'eau ultrapure pendant 3 minutes. La pièce textile est ensuite lavée dans l'eau ultrapure à 80°C (FISONS C1, Thermo Scientific Haake, Karlsruhe, Allemagne) pendant 20 minutes et séchée pendant 104°C pendant 1 heure, et enfin refroidie 30 minutes à température ambiante dans un dessicateur. Le taux de greffage est revêtement hôte est de l'ordre de 29%. La pièce textile A finale est souple, et s'enroule sur elle-même sans altération de sa forme ni détachement du revêtement polymère hôte de CD.

Exemple 4A : Un disque de 11 mm de diamètre découpé dans la pièce textile A obtenue à la fin de l'exemple 1 est plongé dans une solution de chlorhydrate de ropivacaine à 2mg/mL pendant 5 minutes, et ensuite plongé 60 minutes dans 10 ml d'eau ultrapure pour ôter la ropivacaine non adsorbée. La quantité (g) de ropivacaine désorbée par rapport à la masse totale de la pièce textile A revêtue du polymère de CD est de 9,0 +/- 2,5 mg/g.

Exemple 4B : Un disque de 11 mm de diamètre découpé dans la pièce textile A obtenue à la fin de l'exemple 1 est plongé dans une solution de chlorhydrate de ropivacaine à 10 mg/mL pendant 5 minutes, et ensuite plongé 60 minutes dans 10 ml d'eau ultrapure pour ôter la ropivacaine non adsorbée. La quantité (g) de ropivacaine désorbée par rapport à la masse totale de la pièce textile A revêtue du polymère de CD est de 60 mg/g.

Exemple 5A : même protocole que pour l'exemple 4A mais appliqué à l'exemple 2.

Exemple 5B : même protocole que pour l'exemple 4B mais appliqué à l'exemple 2.

Exemple 6A : même protocole que pour l'exemple 4A mais appliqué à l'exemple 3.

Exemple 6B : même protocole que pour l'exemple 4B mais appliqué à l'exemple 3. On observe pour les exemples 5A et 6A, des taux de désorption dans l'eau ultrapure similaires au taux de désorption en ropivacaine dans l'eau ultrapure obtenu pour l'exemple 4A. De même pour les taux de désorption dans l'eau ultrapure des exemples 5B et 6B qui sont similaires au taux de désorption en ropivacaine dans l'eau ultrapure pour l'exemple 4B.

Exemple 7A : Le disque obtenu à l'issu de l'exemple 4A est plongé dans 5 ml d'une solution d'hydroxyde de sodium à 0,1M pendant 5 minutes à 37°C pour hydrolyser complétement le revêtement polymère hôte formé à partir de HPβCD, libérant ainsi totalement la ropivacaine adsorbée. La quantité (g) de ropivacaine désorbée par rapport à la masse totale de la pièce textile A revêtue du polymère de CD est de 5,1 +/- 0,1 mg/g.

Exemple 7B : Le disque obtenu à l'issu de l'exemple 4B est plongé dans 5 ml d'une solution d'hydroxyde de sodium à 0,1M pendant 5 minutes à 37°C pour hydrolyser complétement le revêtement polymère hôte formé à partir de HPβCD, libérant ainsi totalement la ropivacaine adsorbée. La quantité (g) de ropivacaine désorbée par rapport à la masse totale de la pièce textile A revêtue du polymère de CD est de 18,8 +/- 0,1 mg/g. La quantité totale de ropivacaine désorbée dans l'eau et la soude est donc de l'ordre de 78,8 mg/g de support, i.e de la pièce textile A.

Exemple 8A : même protocole que pour l'exemple 7A mais appliqué à l'exemple 5A. La quantité (g) de ropivacaine désorbée par rapport à la masse totale de la pièce textile A revêtue du polymère de CD est de 7,2 +/- 0,5 mg/g.

Exemple 8B : même protocole que pour l'exemple 7B mais appliqué à l'exemple 5B. La quantité (g) de ropivacaine désorbée par rapport à la masse totale de la pièce textile A revêtue du polymère de CD est de 23,1 +/- 1,1 mg/g.

Exemple 9A : même protocole que pour l'exemple 7A mais appliqué à l'exemple 6A. La quantité (g) de ropivacaine désorbée par rapport à la masse totale de la pièce textile A revêtue du polymère de CD est de 9,4 +/- 0,8 mg/g.

Exemple 9B : même protocole que pour l'exemple 7B mais appliqué à l'exemple 6B. La quantité (g) de ropivacaine désorbée par rapport à la masse totale de la pièce textile A revêtue du polymère de CD est de 33,9 +/- 1,1 mg/g.

Exemple 10 : La pièce textile B est imprégnée et foulardée dans une solution aqueuse comprenant HPβCD, un catalyseur (NaH₂PO₂) et CTR, dans les proportions suivantes 8/1/10 dans lesquelles 8, 1 et 10 sont les masses (en grammes) de HPβCD, du catalyseur (NaH₂PO₂) et de CTR, respectivement, dissout dans 100 mL d'eau ultrapure. La pièce textile B imprégnée est placée dans une enceinte de thermofixation (Minithermo^{®}, Roaches, UK) pendant 60 minutes à 160°C. La pièce textile B revêtue est lavée à l'eau distillée puis traitée dans une solution aqueuse comprenant du carbonate de sodium, puis lavée avec de l'eau distillée à une température de l'ordre de 80°C dans un extracteur Soxhlet. Le taux de greffage en revêtement hôte est de l'ordre de 13,6 %.

Exemple 11 : La pièce textile B est imprégnée et foulardée dans une solution aqueuse comprenant HPβCD, un catalyseur (NaH₂PO₂) et CTR, dans les proportions suivantes 12/1,5/15 dans lesquelles 12 ; 1,5 et 15 sont les masses (en grammes) de HPβCD, du catalyseur (NaH₂PO₂) et de CTR, respectivement, dissouts dans 100 mL d'eau ultrapure. La pièce textile B imprégnée est placée dans une enceinte de thermofixation (Minithermo^{®}, Roaches, UK) pendant 60 minutes à 160°C. La pièce textile B revêtue est lavée à l'eau distillée puis traitée dans une solution aqueuse comprenant du carbonate de sodium, puis lavée avec de l'eau distillée à une température de l'ordre de 80°C dans un extracteur Soxhlet. Le taux de greffage en revêtement hôte est de l'ordre de 17,4 %.

Exemple 12 : La pièce textile B est imprégnée et foulardée dans une solution aqueuse comprenant HPβCD, un catalyseur (NaH₂PO₂) et CTR, dans les proportions suivantes 16/2/20 dans lesquelles 16, 2 et 20 sont les masses (en grammes) de HPβCD, du catalyseur (NaH₂PO₂) et de CTR, respectivement, dissouts dans 100 mL d'eau ultrapure. La pièce textile B imprégnée est placée dans une enceinte de thermofixation (Minithermo^{®}, Roaches, UK) pendant 60 minutes à 160°C. La pièce textile B revêtue est lavée à l'eau distillée puis traitée dans une solution aqueuse comprenant du carbonate de sodium, puis lavée avec de l'eau distillée à une température de l'ordre de 80°C dans un extracteur Soxhlet. Le taux de greffage en revêtement hôte est de l'ordre de 27,7 %.

Exemple 13 : Un disque de 11mm dans la pièce textile B de l'exemple 11 subit les protocoles décrits dans les exemples 4A et 7A. Le taux de ropivacaine désorbée dans une solution de soude NaOH 0.1M est de l'ordre de 4 mg/g.

Exemple 14 comparatif : Un disque de 11 mm dans une pièce textile B vierge subit les protocoles décrits dans les exemples 4A et 7A. Le taux de ropivacaine désorbée dans une solution de soude NaOH 0.1M est de l'ordre de 0,5 mg/g (de la pièce textile B). Il convient de noter que le même comportement, à savoir une très faible absorption en ropivacaine, est observé pour la pièce textile A vierge.

Exemple 15 : Un disque de 11 mm dans la pièce textile B revêtue de CD à l'exemple 11 subit les protocoles décrits dans les exemples 4B et 7B (Le quantité de ropivacaine désorbée dans une solution de soude NaOH 0.1M est de l'ordre de 9 mg/g. La quantité totale de ropivacaine désorbée (dans l'eau et la soude) est de l'ordre de 49,4 mg/g de pièce textile B.

Exemple 16 comparatif : Un disque de 11 mm dans une pièce textile B vierge subit les protocoles décrits dans les exemples 4B et 7B. Le taux de ropivacaine désorbé dans une solution de soude NaOH 0.1M est de l'ordre de 2 mg/g. Il convient de noter que le même comportement, à savoir une très faible absorption en ropivacaine, est observé pour la pièce textile A vierge.

La quantité totale de ropivacaine adsorbée (mg/g de support) par la pièce textile A fonctionnalisée (exemple 7B) est augmentée de 60% comparativement à la pièce textile B fonctionnalisée (exemple 15).

Si on considère la quantité totale de ropivacaine par m² de pièce textile, la quantité de ropivacaine adsorbée (mg/m² de support textile) pour la pièce textile B de l'exemple 15 est de l'ordre de 5611 mg/m² contre 3410 mg de ropivacaine par m² de support textile A de l'exemple 7B. Ainsi, bien que la pièce textile A soit 2,6 fois plus légère que la pièce textile B, et que la quantité de ropivacaine adsorbée par m² soit 0,39 fois moins (5,28 g de polymère de CD greffé contre 13,6 g de polymère de CD greffé), la quantité de ropivacaine adsorbée n'est amoindrie que de 0,60 fois.

Cet avantage permet d'obtenir une prothèse souple et offrant des capacités d'absorption en agents actifs améliorées. Ainsi l'utilisation d'un support textile allégé offre un excellent compromis entre i) la quantité de polymère de cyclodextrine immobilisée par m² de pièce textile, ii) la quantité de ropivacaine chargée sur la pièce textile, et iii) la souplesse de la pièce textile

La quantité d'analgésique chargée par la pièce textile A ou B fonctionnalisée par le polymère de CD est supérieure à celle chargée par une pièce textile A ou B vierge, en particulier lorsque la solution de ropivacaine est concentrée à 10 mg/mL.

La présence du polymère de CD permettrait ainsi d'améliorer la quantité de ropivacaine adsorbée physiquement par la pièce textile.

Les pièces textiles selon l'invention ont été testées afin de déterminer leur efficacité sur le traitement de la douleur en se basant sur le modèle de distension colorectale développé par Rousseaux et al (« Lactobacillus acidophilus modulates intestinal pain and induces opioid and cannabinoid receptors.Nat. Med. 13 (2007) 35-37) et Yang et al (« Establishment of model of visceral pain due to colorectal distension and its behavioral assessment in rats. World J.Gastroenterol. 12 (2006) 2781-2784).

Des pièces textiles A, vierges et fonctionnalisées par un revêtement polymère hôte selon l'exemple 3, non chargées en ropivacaine, ont été implantées dans deux groupes de chacun dix rats . L'évaluation de la douleur a été évaluée un jour après implantation jusqu'à 8 jours. Aucun décès post-opératoire n'a été observé.

On observe ainsi un seuil de la douleur faible de 38 mmHg (contre 46 mmHg considéré comme le niveau de base) pour la pièce textile A vierge et la pièce textile A fonctionnalisée selon l'exemple 3 non chargées en ropivacaine, un jour après implantation. Cette tolérance à la douleur faible révèle la douleur viscérale induite par la chirurgie et l'absence d'un effet analgésique des prothèses implantées décrites ci-dessus. Ce seuil a persisté jusqu'au huitième jour puisqu'il est de 42,7 mmHg +/- 1,7 mmHg.

Des pièces textiles A, vierges et fonctionnalisées par un revêtement polymère hôte selon l'exemple 3, chargé en ropivacaine (10 mg/g), ont été implantées dans deux groupes de chacun 10 rats. L'évaluation de la douleur a été évaluée un jour après implantation jusqu'à 8 jours. Aucun décès post-opératoire n'a été observé.

On observe ainsi un seuil de la douleur amélioré à 46 mmHg considéré pour la pièce textile A vierge chargée en ropivacaine (10 mg/g) sur les deux premiers jours après implantation. Un effet de soulagement de la douleur significatif a été observé pour l'exemple 3, chargé en ropivacaine selon la même concentration (10 mg/g), durant les quatre premiers jours après implantation, le seuil étant de 53 +/- 1,71 mmHg démontrant ainsi la contribution du polymère de CD dans la libération de la ropivacaine. A quantité de ropivacaine égale, la pièce textile A vierge et la pièce textile A de l'exemple 3 n'ont pas le même comportement. Le dispositif implantable selon l'exemple 3 est plus efficace que la pièce textile A vierge en termes de durée et d'intensité de la tolérance à la douleur.

## Revendications

1. Dispositif implantable comprenant une pièce textile au moins partiellement revêtue d'un revêtement polymère hôte, ledit revêtement polymère hôte comprend un polymère de cyclodextrine(s) et/ou dérivés de cyclodextrine(s) et/ou de complexe(s) d'inclusion de cyclodextrine et/ou de complexe(s) d'inclusion de dérivés de cyclodextrines, **caractérisé en ce que** ladite pièce textile comprend des fils multifilamentaires et/ou des fils filés de fibres et **en ce qu'**au moins une partie des filaments et/ou des fibres ont chacun(e) un diamètre inférieur ou égal à 25 micromètres.

2. Dispositif implantable selon la revendication 1, **caractérisé en ce que** le rapport de la masse du revêtement hôte sur la masse totale de la pièce textile revêtue dudit revêtement polymère hôte est supérieur ou égal à 10%, de préférence supérieur ou égal à 12%, encore de préférence supérieur ou égal à 15%.

3. Dispositif implantable selon l'une ou l'autre des revendications 1 et 2, **caractérisé en ce que** la pièce textile a une masse surfacique inférieure ou égale à 120 g/m², de préférence inférieure ou égale à 80 g/m², encore de préférence inférieure ou égale à 70 g/m², encore de préférence inférieure ou égale à 50 g/m².

4. Dispositif implantable selon l'une quelconque des revendications 1 à 3, **caractérisé en ce que** la pièce textile comprend des fils multifilamentaires et/ou des fils filés de fibres dont le titre des filaments et/ou des fibres est inférieur ou égal à 10 dtex, de préférence inférieur ou égal à 7 dtex, encore de préférence inférieur ou égal à 4 dtex.

5. Dispositif implantable selon l'une quelconque des revendications 1 à 4, **caractérisé en ce que** la pièce textile est une pièce tricotée, notamment à mailles jetées.

6. Dispositif implantable selon l'une quelconque des revendications 1 à 5, **caractérisé en ce que** ledit dispositif est choisi dans la liste constituée d'une prothèse pour le traitement d'une hernie dans la région abdominale, notamment d'une hernie inguinale, fémorale, ombilicale ou épigastrique ; d'un ligament ; d'une prothèse en traitement d'un prolapsus d'au moins un organe pelvien, d'une prothèse de traitement des dysfonctionnements érectiles masculins ; d'une prothèse pour le traitement du rachis, notamment d'une prothèse inter-vertébrale ; de préférence d'une prothèse pour le traitement d'une hernie.

7. Dispositif implantable selon l'une quelconque des revendications 1 à 6, **caractérisé en ce que** les fils multifilamentaires et/ou les fils filés de fibres sont dans au moins un ou plusieurs polymère(s) choisi(s) dans la liste I constituée par : les polyoléfines; les polymères à base de polyoxyde d'éthylène (PEO) ; les copolymères à blocs à base de polyoxyde de propylène et d'oxyde d'éthylène, notamment de type dibloc ou tribloc; les polyesters ; les polyamides, et/ou dans la liste II constituée par : les (co)polymères d'acide lactique, issu(s) de la polymérisation au moins du L Lactide et/ou au moins du D Lactide et/ou au moins du mésolactide ; les polymères issus de la polymérisation au moins du glycolide; les copolymères issus de la polymérisation d'au moins du lactide et d'au moins du glycolide; les (co)polymères à blocs à base de polylactide et de polyéthylèneglycol; ou un mélange de ces derniers.

8. Dispositif implantable selon l'une quelconque des revendications 1 à 7, **caractérisé en ce que** le revêtement polymère hôte comprend au moins un agent fonctionnel invité choisi parmi la liste comprenant : les antibiotiques ; de préférence la ciprofloxacine ; les agents anti-inflammatoires ; les anticoagulants ; les anti-thrombogéniques ; les agents anti-mitotiques ; les agents anti-prolifération ; les agents anti-adhésion ; les agents anti-migration ; les promoteurs d'adhésion cellulaire ; les facteurs de croissance ; les molécules antiparasitaires ; les hormones ; les antifongiques ; les molécules antimicrobiennes ; les antiseptiques ; les agents analgésiques tels que la ropivacaine, la bupivacaine, la lidocaine, la lévobupivacaine, de préférence la ropivacaine.

9. Dispositif implantable selon l'une quelconque des revendications 1 à 8, **caractérisé en ce que** la pièce textile comprend des jours, en particulier comprend des jours ayant au moins une dimension supérieure ou égale à 1 mm.

10. Dispositif implantable selon l'une quelconque des revendications 1 à 9, **caractérisé en ce que** la pièce textile est choisie dans la liste constituée par: les tricots à mailles jetées ou à mailles cueillies; les tissus, les tresses, les éléments retordus, ou leur combinaison.

11. Procédé de fabrication d'un dispositif implantable, de préférence selon l'une quelconque des revendications 1 à 10, comprenant les étapes suivantes :
- (i) fourniture d'une pièce textile comprenant des fils multifilamentaires et /ou des fils filés de fibres, au moins une partie des filaments et/ou des fibres ont chacun(e) un diamètre inférieur à 25 micromètres ;
- (ii) application d'une solution aqueuse comprenant:
- au moins une cyclodextrine et/ou au moins un dérivé de cyclodextrine et/ou au moins un complexe d'inclusion de cyclodextrine et/ou au moins un complexe d'inclusion de dérivé de cyclodextrine ;
- au moins un acide (poly)carboxylique et/ou son anhydride d'acide (poly)carboxylique,
- et éventuellement au moins un catalyseur,
sur au moins une partie des fils multifilamentaires et/ou des fils filés de fibres ;
- (iii) application d'un traitement thermique à la pièce textile obtenue après l'étape (ii) afin de former un revêtement hôte comprenant un polymère de cyclodextrine(s) et/ou dérivés de cyclodextrine(s) et/ou de complexe(s) d'inclusion de cyclodextrine et/ou de complexe(s) d'inclusion dérivés de cyclodextrines ;
- obtention du dispositif implantable.

12. Procédé de fabrication selon la revendication 11, **caractérisé en ce qu'**il comprend une étape de lavage (iv) de la pièce textile après l'étape (iii) au-cours de laquelle la pièce textile est lavée avec de l'eau ou une solution aqueuse ayant une température supérieure ou égale à 45°C, notamment supérieure ou égale à 70°C.

13. Procédé de fabrication selon l'une ou l'autre des revendications 11 et 12, **caractérisé en ce qu'**il comprend une étape de neutralisation de l'acidité résiduelle du revêtement hôte, de préférence on applique une solution basique, en particulier une solution de carbonate de sodium ou d'hydrogénocarbonate de sodium, à la pièce textile obtenue après l'étape (iii).

## Patentansprüche

1. Implantierbare Vorrichtung, umfassend ein Textilstück, das zumindest teilweise mit einer Wirtspolymerbeschichtung beschichtet ist, wobei die Wirtspolymerbeschichtung ein Polymer von Cyclodextrin(en) und/oder Derivate von Cyclodextrin(en) und/oder (einen) Einschlusskomplex(e) von Cyclodextrin und/oder (einen) Einschlusskomplex(e) von Cyclodextrinderivaten umfasst, **dadurch gekennzeichnet, dass** das Textilstück Multifilamentgarne und/oder gesponnene Fasergarne umfasst, und dadurch, dass mindestens ein Teil der Filamente und/oder der Fasern jeweils einen Durchmesser aufweist, der kleiner oder gleich 25 Mikrometer ist.

2. Implantierbare Vorrichtung nach Anspruch 1, **dadurch gekennzeichnet, dass** das Verhältnis der Masse der Wirtsbeschichtung zu der Gesamtmasse des mit der Wirtspolymerbeschichtung beschichteten Textilstücks größer oder gleich 10 %, vorzugsweise größer oder gleich 12 %, insbesondere größer oder gleich 15 % ist.

3. Implantierbare Vorrichtung nach einem der Ansprüche 1 und 2, **dadurch gekennzeichnet, dass** das Textilstück ein Flächengewicht aufweist, das kleiner oder gleich 120 g/m², vorzugsweise kleiner oder gleich 80 g/m², insbesondere kleiner oder gleich 70 g/m², insbesondere kleiner oder gleich 50 g/m² ist.

4. Implantierbare Vorrichtung nach einem der Ansprüche 1 bis 3, **dadurch gekennzeichnet, dass** das Textilstück Multifilamentgarne und/oder gesponnene Fasergarne umfasst, deren Filament- und/oder Fasertiter kleiner oder gleich 10 dtex, vorzugsweise kleiner oder gleich 7 dtex, insbesondere kleiner oder gleich 4 dtex ist.

5. Implantierbare Vorrichtung nach einem der Ansprüche 1 bis 4, **dadurch gekennzeichnet, dass** das Textilstück ein Gestrick, besonders mit gewirkten Maschen, ist.

6. Implantierbare Vorrichtung nach einem der Ansprüche 1 bis 5, **dadurch gekennzeichnet, dass** die Vorrichtung aus der Liste ausgewählt ist, die besteht aus: einer Prothese zur Behandlung einer Hernie im Bauchbereich, besonders eines Leistenbruchs, einer Femoralhernie, eines Nabelbruchs oder einer epigastrischen Hernie, einem Band, einer Prothese zur Behandlung eines Vorfalls von mindestens einem Unterleibsorgan, einer Prothese zur Behandlung erektiler Dysfunktionen bei Männern, einer Prothese zur Behandlung der Wirbelsäule, besonders einer Intervertebralprothese, vorzugsweise einer Prothese zur Behandlung einer Hernie.

7. Implantierbare Vorrichtung nach einem der Ansprüche 1 bis 6, **dadurch gekennzeichnet, dass** die Multifilamentgarne und/oder die gesponnenen Fasergarne in mindestens einem oder mehreren Polymer(en) vorliegen, das bzw. die aus der Liste I ausgewählt ist bzw. sind, die besteht aus: den Polyolefinen, den Polymeren auf Polyethylenoxidbasis (PEO), den Blockcopolymeren auf Polypropylenoxid- und Ethylenoxidbasis, besonders vom Diblock- oder Triblocktyp, den Polyestern, den Polyamiden, und/oder aus der Liste II, die besteht aus: den Milchsäure(co)polymeren, die zumindest aus der Polymerisation von L-Lactid und/oder zumindest von D-Lactid und/oder zumindest von meso-Lactid hervorgehen, den Polymeren, die zumindest aus der Polymerisation von Glycolid hervorgehen, den Copolymeren, die zumindest aus der Polymerisation von Lactid und zumindest von Glycolid hervorgehen, den Block(co)polymeren auf Polylactid- und Polyethylenglykolbasis, oder einem Gemisch der Letztgenannten.

8. Implantierbare Vorrichtung nach einem der Ansprüche 1 bis 7, **dadurch gekennzeichnet, dass** die Wirtspolymerbeschichtung mindestens ein erwünschtes funktionelles Mittel umfasst, das aus der Liste ausgewählt ist, umfassend: die Antibiotika, vorzugsweise Ciprofloxacin, die Entzündungshemmer, die Antikoagulanzien, die Antithrombosemittel, die Antimitotika, die antiproliferativen Mittel, die Antihaftmittel, die Antimigrationsmittel, die Zelladhäsionspromotoren, die Wachstumsfaktoren, die antiparasitären Moleküle, die Hormone, die Antipilzmittel, die antimikrobiellen Moleküle, die Antiseptika, die Analgetika, wie das Ropivacain, das Bupivacain, das Lidocain, das Levobupivacain, vorzugsweise das Ropivacain.

9. Implantierbare Vorrichtung nach einem der Ansprüche 1 bis 8, **dadurch gekennzeichnet, dass** das Textilstück Hohlnahten umfasst, insbesondere Hohlnahten umfasst, die mindestens eine Abmessung aufweisen, die größer oder gleich 1 mm ist.

10. Implantierbare Vorrichtung nach einem der Ansprüche 1 bis 9, **dadurch gekennzeichnet, dass** das Textilstück aus der Liste ausgewählt ist, die besteht aus: den Gewirken mit gewirkten Maschen oder mit Kuliermaschen, den Geweben, den Geflechten, den verzwirnten Elementen oder ihrer Kombination.

11. Herstellungsverfahren einer implantierbaren Vorrichtung, vorzugsweise nach einem der Ansprüche 1 bis 10, umfassend die folgenden Schritte:
- (i) Bereitstellen eines Textilstücks, umfassend Multifilamentgarne und/oder gesponnene Fasergarne, wobei mindestens ein Teil der Filamente und/oder der Fasern jeweils einen Durchmesser aufweist, der kleiner oder gleich 25 Mikrometer ist,
- (ii) Anwenden einer wässrigen Lösung, umfassend:
- mindestens ein Cyclodextrin und/oder mindestens ein Cyclodextrinderivat und/oder mindestens einen Einschlusskomplex von Cyclodextrin und/oder mindestens einen Einschlusskomplex eines Cyclodextrinderivats,
- mindestens eine (Poly)-Carbonsäure und/oder ihr (Poly)-Carbonsäureanhydrid,
- und gegebenenfalls mindestens einen Katalysator,
zumindest auf einen Teil der Multifilamentgarne und/oder der gesponnenen Fasergarne,
- (iii) Anwenden einer Wärmebehandlung auf das nach Schritt (ii) erhaltene Textilstück, um eine Wirtsbeschichtung zu bilden, die ein Polymer von Cyclodextrin(en) und/oder Derivate von Cyclodextrin(en) und/oder (einen) Einschlusskomplex(e) von Cyclodextrin und/oder (einen) Einschlusskomplex(e) von Cyclodextrinderivaten umfasst,
- Erhalten der implantierbaren Vorrichtung.

12. Herstellungsverfahren nach Anspruch 11, **dadurch gekennzeichnet, dass** es einen Waschschritt (iv) des Textilstücks nach Schritt (iii) umfasst, in dessen Verlauf das Textilstück mit Wasser oder einer wässrigen Lösung gewaschen wird, das bzw. die eine Temperatur größer oder gleich 45 °C, besonders größer oder gleich 70 °C, aufweist.

13. Herstellungsverfahren nach einem der Ansprüche 11 und 12, **dadurch gekennzeichnet, dass** es einen Schritt zur Neutralisierung des Restsäuregehalts der Wirtsbeschichtung umfasst, wobei vorzugsweise eine basische Lösung, insbesondere eine Lösung von Natriumcarbonat oder Natriumhydrogencarbonat, auf das nach Schritt (iii) erhaltene Textilstück angewendet wird.

## Claims

1. An implantable device comprising a textile component at least partially coated with a host polymer coating, said host polymer coating comprising a polymer of cyclodextrin(s) and/or derivatives of cyclodextrin(s) and/or inclusion complex(es) of cyclodextrin and/or inclusion complex(es) of cyclodextrin derivatives, **characterized in that** said textile component comprises multifilament yarns and/or spun yarns made of fibers and **in that** at least a part of the filaments and/or fibers each have a diameter of less than or equal to 25 micrometers.

2. The implantable device as claimed in claim 1, **characterized in that** the ratio of the mass of the host coating to the total mass of the textile component coated with said host polymer coating is greater than or equal to 10%, preferably greater than or equal to 12%, more preferably greater than or equal to 15%.

3. The implantable device as claimed in either one of claims 1 and 2, **characterized in that** the textile component has an surface density of less than or equal to 120 g/m², preferably less than or equal to 80 g/m², more preferably less than or equal to 70 g/m², more preferably less than or equal to 50 g/m².

4. The implantable device as claimed in any one of claims 1 to 3, **characterized in that** the textile component comprises multifilament yarns and/or spun yarns made of fibers whose titer of filaments and/or fibers is less than or equal to 10 dtex, preferably less than or equal to 7 dtex, more preferably less than or equal to 4 dtex.

5. The implantable device as claimed in any one of claims 1 to 4, **characterized in that** the textile component is a knitted component, in particular a warp knit component.

6. The implantable device as claimed in any one of claims 1 to 5, **characterized in that** said device is selected from the list consisting of a prosthesis for the treatment of a hernia in the abdominal region, in particular an inguinal, femoral, umbilical or epigastric hernia; a ligament; a prosthesis for the treatment of a prolapse of at least one pelvic organ; a prosthesis for the treatment of male erectile dysfunction; a prosthesis for the treatment of the spine, in particular an intervertebral prosthesis; preferably a prosthesis for the treatment of a hernia.

7. The implantable device as claimed in any one of claims 1 to 6, **characterized in that** the multifilament yarns and/or the spun yarns made of fibers are made of at least one or more polymer(s) chosen from List I consisting of: polyolefins; polymers based on polyethylene oxide (PEO); block copolymers based on polypropylene oxide and ethylene oxide, in particular of the diblock or triblock type; polyesters; polyamides, and/or from List II consisting of: (co)polymers of lactic acid, derived from the polymerization of at least L-lactide and/or at least D-lactide and/or at least meso-lactide; polymers derived from the polymerization of at least glycolide; copolymers derived from the polymerization of at least lactide and at least glycolide; block (co)polymers based on polylactide and polyethylene glycol; or a mixture thereof.

8. The implantable device as claimed in any one of claims 1 to 7, **characterized in that** the host polymer coating comprises at least one guest functional agent selected from the list comprising: antibiotics; preferably ciprofloxacin; antiinflammatory agents; anticoagulants; antithrombogenic agents; anti-mitotic agents; anti-proliferation agents; anti-adhesion agents; anti-migration agents; cell adhesion promoters; growth factors; antiparasitic molecules; hormones; antifungals; antimicrobial molecules; antiseptics; analgesic agents such as ropivacaine, bupivacaine, lidocaine, levobupivacaine, preferably ropivacaine.

9. The implantable device as claimed in any one of claims 1 to 8, **characterized in that** the textile component comprises openings, in particular comprises openings having at least one dimension greater than or equal to 1 mm.

10. The implantable device as claimed in any of claims 1 to 9, **characterized in that** the textile component is selected from the list consisting of: warp or weft knit material; woven material, braids, twisted elements, or a combination thereof.

11. A process for manufacturing an implantable device, preferably as claimed in any one of claims 1 to 10, comprising the following steps:
- (i) providing a textile component comprising multifilament yarns and/or spun yarns made of fibers, at least a part of the filaments and/or fibers each having a diameter of less than 25 micrometers;
- (ii) applying an aqueous solution comprising:
- at least one cyclodextrin and/or at least one cyclodextrin derivative and/or at least one inclusion complex of cyclodextrin and/or at least one inclusion complex of cyclodextrin derivative;
- at least one (poly)carboxylic acid and/or its (poly)carboxylic acid anhydride,
- and optionally at least one catalyst,
on at least a part of the multifilament yarns and/or spun yarn made of fibers;
- (iii) applying a heat treatment to the textile component obtained after step (ii) in order to form a host coating comprising a polymer of cyclodextrin(s) and/or derivatives of cyclodextrin(s) and/or inclusion complex(es) of cyclodextrin and/or inclusion complex(es) derivatives of cyclodextrins;
- obtaining the implantable device.

12. The manufacturing process as claimed in claim 11, **characterized in that** it comprises a step of washing (iv) of the textile component after step (iii) during which the textile component is washed with water or an aqueous solution having a temperature greater than or equal to 45°C, in particular greater than or equal to 70°C.

13. The manufacturing process as claimed in either of claims 11 and 12, **characterized in that** it comprises a step of neutralizing the residual acidity of the host coating, preferably a basic solution, in particular a solution of sodium carbonate or sodium hydrogen carbonate, is applied to the textile component obtained after step (iii).
